# EUROPEAN PATENT SPECIFICATION

(11) **EP 1 091 686 B1**
(45) Date of publication and mention of the grant of the patent: **21.05.2003**
(21) Application number: 99940409.8
(22) Date of filing: 06.09.1999
(51) Int. Cl.: A61B 5/0285, A61B 5/024, A61B 7/00

(54) **DEVICE OF DETERMINING THE ELASTICITY OF THE ARTERIAL WALL**
VORRICHTUNG ZUR BESTIMMUNG DER ELASTIZITÄT EINER ARTERIENWAND
DISPOSITIF DE DETERMINATION DE L'ELASTICITE DE LA PAROI ARTERIELLE

(30) Priority: 09.09.1998 GR 98100336
(43) Date of publication of application: 18.04.2001
(73) Proprietor: Marcoyannopoulou Fojas, Helen, 106 75 Athens (GR)
(72) Inventor: Marcoyannopoulou Fojas, Helen, 106 75 Athens (GR)
(86) International application number: PCT/GR99/00032
(87) International publication number: WO 00/013584

(56) References cited:
- EP-A- 0 472 464
- E.R. NYE: "The effect of blood pressure alteration on the pulse wave velocity" BRITISH HEART JOURNAL, vol. 26, 1964, pages 261-265, XP002099464

## Description

The present invention relates to medical technology but more particularly, to a device of determining the elasticity of the arterial wall.

### BACKGROUND OF THE INVENTION.

I have been working on the oscillographic method of recording the arterial pulse ever since I did my research work on my thesis "Aortic Stenosis by Indirect Oscillography" for my Degree of Doctor of Medicine from the University of Geneva. This continued to be my field of interest for many years while working as Research Assistant to Professor Pierre Duchosal of the Department of Cardiology, University of Geneva and then with the famous Director of the Institute of Cardiology of London, Dr. Paul Wood. The problem then for some years was to record an arterial pulse wave, which very closely resembled the intra- arterial pulse with the use of a high frequency oscillograph. By repeated trials, I was able to design an attachment to the pick-up of the microphone of the NEP pulse recording unit (Type A-643) of Sanborn. This is described below. Having devised this attachment to record the desired arterial pulse and using a method which I also designed and described below, I planned a research work on normal and hypercholesterolaemic subjects at the Brompton Hospital in London. For this, I was awarded the Alice Hamilton Fellowship for research on haemodynamics for two years by the US based International Federation of University Women - a lone award given annually to one female University graduate chosen from candidates from over the world and this was the first time that Greece won the award. In the recent 2^{nd} International Symposium on Diseases of the Aorta held in Athens, Greece in April 1996, great interest was shown on the elasticity of the arterial wall as shown by the number of research papers read but with the use of more sophisticated invasive and non-invasive methods. In the discussions wherein I participated, it came out that my method, which was much simpler and least expensive had comparable results. I was invited to present my work on normal subjects of different age groups in the 20^{th} Annual Congress of the South African Cardiological Society in October 1996. Communication with a top world authority on pulse wave velocity, Professor Michael F. O'Rourke of the University of South Wales, Australia, also confirmed the consistency of this method compared with more sophisticated techniques (Doppler Flow and tonometry) and giving the same results.

### BRIEF SUMMARY OF THE INVENTION.

It is the primary object of the present invention to provide for indirectly determining the elasticity of the arterial wall. The device may be used in a method which is based on the speed of the transmission of the pulse wave through the arteries. The more elastic the arteries, the slower will be the transmission time and as the elasticity decreases, the faster will be the transmission time. Indirect oscillography is the technique used to measure the speed of the flow of blood as indicated by the pulse wave velocity. The time interval for the pulse wave to be recorded between two points in the arterial system can be determined by recording the pulse in a "central artery" and a "peripheral artery" which in my method are the left external carotid and left dorsalis pedis arteries, respectively. These arteries are well exposed, easily located and the distance between them is long enough so that a longer segment is measured and the statistical margin of error is less.

The subjects used in this study are within a specific height range. It is essential that the pulses recorded resemble as closely as possible the intra-arterial pulse and this has been achieved by a device as defined in claim 1. A device according to the preamble of claim 1 is known from EP-A-0 472 464.

### BRIEF DESCRIPTION OF FIGURES.

FIGURE 1/4 - Perspective view of the device used in the invention.
FIGURE 2/4 - Recording of the pulse pattern on the oscilloscope screen of the left carotid and left dorsalis arteries .
FIGURE 3/4 - Figure of the location of the attachments of electrodes of the ECG placed on a patient.
FIGURE 4/4 - Recording of the left carotid and left dorsalis pedis arteries pulse wave simultaneously with the Std. Lead II of the ECG.

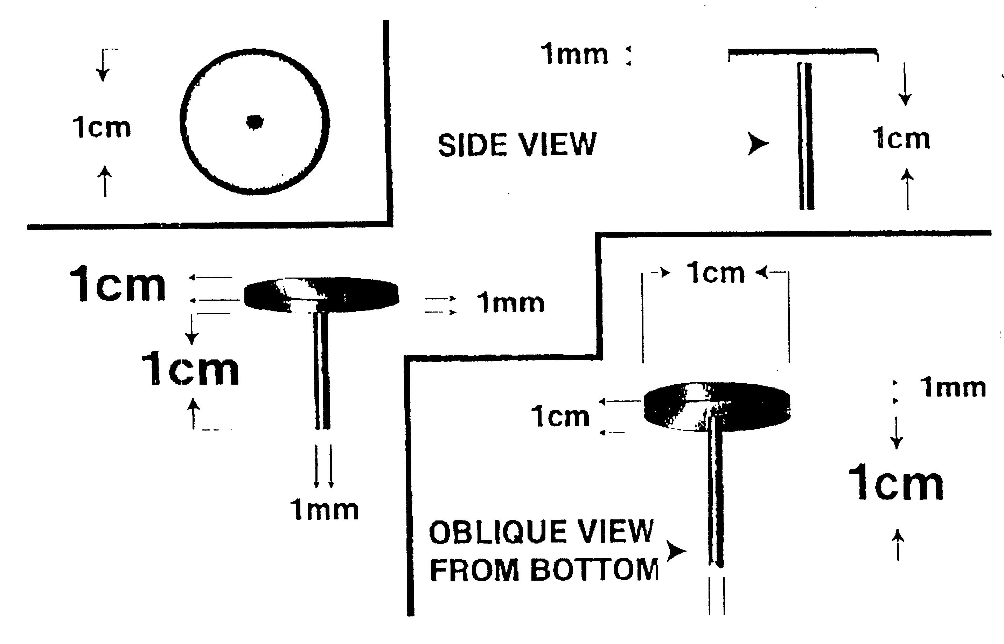

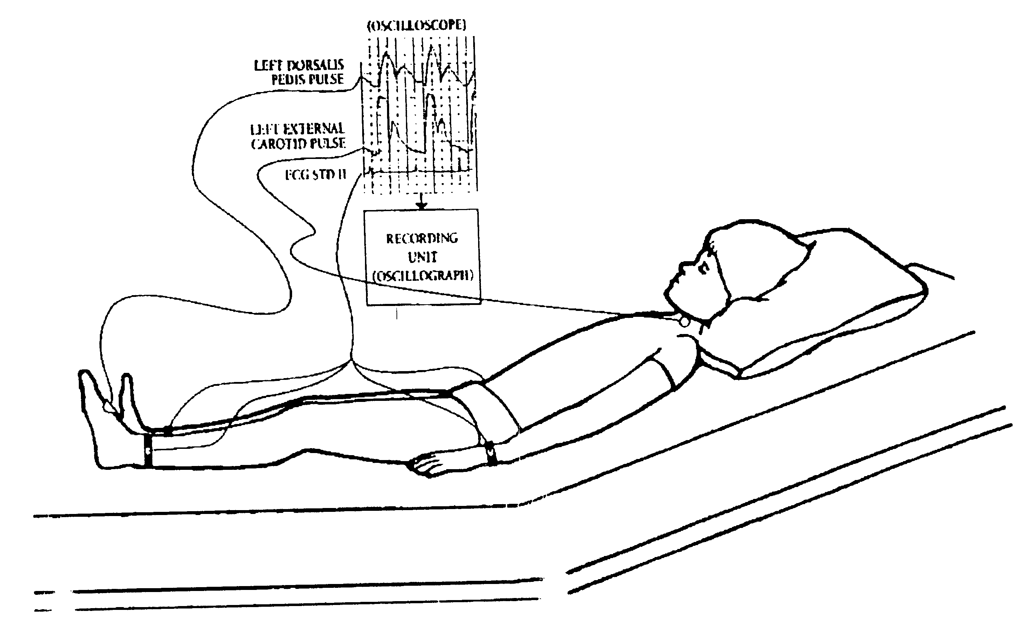

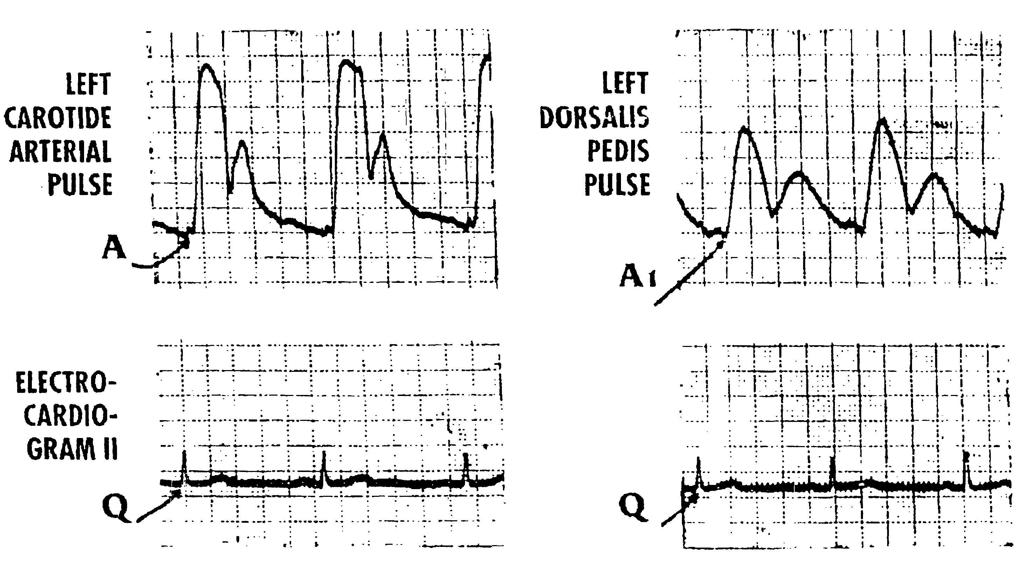

Referring to Fig. 1/4, the device used in the present invention is a circular metallic plate welded to the center of the pick-up microphone of a high frequency oscillograph and is attached on a patient as shown in Fig. 3/4.

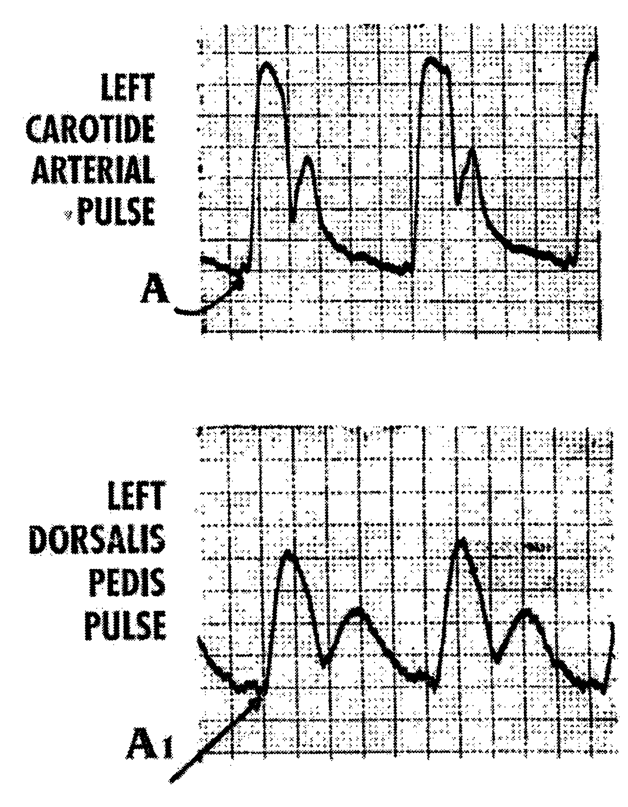

With this attachment placed over the arteries, nice pulses of the left external carotid and left dorsalis pedis arteries are obtained (Fig.2/4). With the subject in the semirecumbent position and the surrounding room temperature kept as constant as possible, the ECG (Std. II) is recorded simultaneously with the left external carotid artery and the left dorsalis pedis arterial pulses (Fig.3/4). Recordings were made in some subjects at intervals of six, twelve and eighteen months and the results were the same. Q is the start of the isometric ventricular contraction, A - the foot point of the anacrotic arterial pulse (left external carotid artery), and A1 the foot point of the anacrotic arterial pulse (left dorsalis pedis artery) (Fig.4/4). So, the difference between QA1 and QA is a measure of the time interval between the instant that the pulse is recorded from the "central artery", i.e., the left external carotid artery and the instant that the same pulse is recorded from the "peripheral artery, i.e., the left dorsalis pedis artery (Fig. 4/4). This method is simple, inexpensive, reproducible and non-invasive. Its results are similar to more sophisticated and expensive methods. It is very applicable for mass screening of big populations (normal and high-risk subjects) especially in developing countries.

## Claims

1. A microphone pick-up attachment for determining the elasticity of the arterial wall, the attachment comprising,
- a circular metallic plate having a centre, and
- a metallic rod having first end welded to said plate centre and said second end for connecting to the centre of a pick-up microphone,
**characterised in that** said circular metallic plate has a diameter of 1 cm and said metallic rod has a length of 2 cm and a diameter of 1 mm.

2. An attachment according to claim 1 further comprising a microphone pick-up welded to said second rod end.

3. An attachment according to claim 2 further comprising a high frequency oscilloscope for simultaneously recording Std. lead II of an ECG and arterial pulses when said attachment is placed on the skin over the left external carotid and the left dorsalis pedis arteries, so as to determine differences between the time the blood travels from the central artery to the peripheral artery.

## Patentansprüche

1. Einbauteil eines Sondermikrophons, zur Feststellung der Elastizität der Arterienwand. Der Einbauteil umfaßt:
- eine runde Metallplatte mit einer Mitte, und
- eine Metallbarre (Stange), deren eine Ecke (Rand) mit der Mitte der o.g Platte, und die o.g zweite Ecke mit der Mitte eines Mikrofons verschweißt ist, zeichnet sich dadurch aus, daß diese Metallplatte, eine Durchmesser von 1 cm, bzw. die o.g Metallplatte eine Länge von 2 cm und Durchmesser von 1 mm aufweist.

2. Ein Zusatzteil It. Anspruch 1, das noch ein Sondermikrofon umfaßt, das in die o.g zweite Ecke des Barrens verschweißt ist.

3. Ein Zusatzteil It. Anspruch 2, der noch ein Oszilloskop für die simultane Schallaufnahme Std. Stift II eines ECG und Arterienpulse beinhaltet, wenn der Zusatzteil auf der Haut, und zwar über der linken Halsschlagader und den li. hinteren Beinarterien aufgestellt wird, so daß die Unterschiede der Zeitspannen, in denen das Blut aus der Zentral- in die Peripherarterie fließt, ermittelt werden.

## Revendications

1. Un accessoire de microphone pour déterminer l'élasticité de la paroi artérielle, cet accessoire comprenant :
- une plaque métallique circulaire ayant un centre, et
- une tige métallique dont la première extrémité est soudée au centre de la plaque susmentionné et dont la seconde extrémité est destinée à être reliée au centre d'un microphone,
**caractérisé par** la plaque métallique circulaire susmentionnée d'un diamètre de 1cm et par la tige métallique précitée d'une longueur de 2cm et d'un diamètre de 1mm.

2. Un accessoire, conformément à la revendication 1, comprenant en outre un microphone soudé à ladite seconde extrémité de la tige.

3. Un accessoire, conformément à la revendication 2, comprenant en outre un oscilloscope à haute fréquence pour enregistrer simultanément la dérivation standard II d'un ECG et les pulsations artérielles quand ledit accessoire est placé sur la peau au niveau de la carotide externe gauche et de l'artère pédieuse gauche, afin de déterminer les différences de temps mis par le sang pour se rendre de l'artère centrale à l'artère périphérique.
